# EUROPEAN PATENT SPECIFICATION

(11) **EP 4 472 546 B1**
(45) Date of publication and mention of the grant of the patent: **29.07.2026**
(21) Application number: 23706113.0
(22) Date of filing: 22.01.2023
(51) Int. Cl.: A61B 34/20, A61B 90/00, A61B 34/30, A61B 90/98

(54) **SYSTEM FOR TRACKING MOVEMENT OF AN ANATOMICAL ELEMENT**
SYSTEM ZUR VERFOLGUNG DER BEWEGUNG EINES ANATOMISCHEN ELEMENTS
SYSTÈME DE SUIVI DU MOUVEMENT D'UN ÉLÉMENT ANATOMIQUE

(30) Priority: 02.02.2022 US 202217590905
(43) Date of publication of application: 11.12.2024
(73) Proprietor: Mazor Robotics Ltd., 3079830 Caesarea (IL)
(72) Inventor: SANDELSON, Adi, 3079830 Caesarea (IL); SEEMANN, Ziv, 3079830 Caesarea (IL); DORI, Nimrod, 3079830 Caesarea (IL)
(74) Representative: Viering, Jentschura & Partner mbB Patent- und Rechtsanwälte
(86) International application number: PCT/IL2023/050069
(87) International publication number: WO 2023/148716

(56) References cited:
- US-A1- 2016 022 176
- US-A1- 2018 185 113
- US-A1- 2019 357 986

## Description

### BACKGROUND

The present disclosure is generally directed to tracking an anatomical element, and relates more particularly to a system for tracking an anatomical element according to the preamble of claim 1.

Surgical robots may assist a surgeon or other medical provider in carrying out a surgical procedure, or may complete one or more surgical procedures autonomously. Imaging may be used by a medical provider for diagnostic and/or therapeutic purposes. Patient anatomy can change over time, particularly following placement of a medical implant in the patient anatomy.

From US 2019/357986 A1 a system for tracking an anatomical element according to the preamble of claim 1 is known. From US 2016/022176 A1 a further system for tracking an anatomical element is known.

### BRIEF SUMMARY

The object of the present invention is providing an improved system for tracking an anatomical element. This object is solved by a system for tracking an anatomical element according to claim 1. Further embodiments are subject of the dependent claims. Example aspects of the present disclosure include:

A system for tracking movement of an anatomical element according to at least one embodiment of the present disclosure comprises a marker coupled to the anatomical element; a processor; and a memory storing data for processing by the processor, the data, when processed, causes the processor to: track the marker; detect a movement of the marker; and determine a pose of the marker based on the movement.

Any of the aspects herein, wherein the marker comprises at least one of an optical marker, an electromagnetic tracker, a radio-frequency identification tracker, a magnetic marker, a light emitting diode, or an infrared light emitting diode.

Any of the aspects herein, wherein the marker is integrated with a surgical implant, the surgical implant attached to the anatomical element.

Any of the aspects herein, wherein the surgical implant is a rod.

Any of the aspects herein, wherein the anatomical element is a vertebra and the marker is releasably secured to a screw head of a screw embedded in the vertebra.

Any of the aspects herein, wherein the screw head comprises a threaded cavity configured to receive a threaded protrusion of the marker.

Any of the aspects herein, wherein the memory stores further data for processing by the processor that, when processed, causes the processor to: compare the pose of the marker to a predetermined pose; and validate the pose of the marker when the pose substantially matches the predetermined pose.

Any of the aspects herein, wherein the memory stores further data for processing by the processor that, when processed, causes the processor to: receive a first image of the anatomical element, the first image obtained preoperatively; obtain a second image from an imaging device, the second image depicting the marker and the anatomical element; and update the first image of the anatomical element based on the second image of the anatomical element and the detected movement.

Any of the aspects herein, wherein the pose comprises at least one of a first pose of the marker and a second pose of the marker.

Any of the aspects herein, wherein detecting movement of the marker comprises comparing the first pose of the marker and the second pose of the marker.

Any of the aspects herein, wherein the pose is obtained from at least one of a navigation system configured to track a pose of the marker or a robotic arm orienting the marker, the robotic arm comprising at least one sensor for sensing a pose of the robotic arm.

A system for tracking movement of an anatomical element according to at least one embodiment of the present disclosure comprises a marker coupled to the anatomical element; a navigation system configured to track a pose of the marker; a processor; and a memory storing data for processing by the processor, the data, when processed, causing the processor to: receive at least a first pose and a second pose of the marker from the navigation system, the second pose received after the first pose; compare the first pose and the second pose; detect a movement of the marker based on the comparison, the movement detected when the second pose does not match the first pose; and generate a notification when the movement meets or exceeds at least one of a movement threshold or a position threshold.

Any of the aspects herein, wherein the memory stores further data for processing by the processor that, when processed, causes the processor to: compare the second pose to a predetermined pose; and validate the second pose when the second pose substantially matches the predetermined pose.

Any of the aspects herein, wherein the memory stores further data for processing by the processor that, when processed, causes the processor to: receive a three-dimensional representation of the anatomical element; and update the three-dimensional representation of the anatomical element based on the detected movement.

Any of the aspects herein, wherein the anatomical element comprises a vertebra.

Any of the aspects herein, wherein the marker comprises at least one of an optical marker, a magnetic marker, an electromagnetic tracker, a radio-frequency identification tracker, a light emitting diode, and an infrared light emitting diode.

Any of the aspects herein, wherein the marker is integrated with a surgical implant that is attached to the anatomical element.

Any of the aspects herein, wherein the marker is releasably secured to a screw head of a screw embedded in the anatomical element.

Any of the aspects herein, wherein the screw head comprises a threaded cavity configured to receive a threaded protrusion of the marker.

A system for tracking movement of an anatomical element according to at least one embodiment of the present disclosure comprises a marker coupled to the anatomical element; a processor; and a memory storing data for processing by the processor, the data, when processed, causing the processor to: receive a first pose of the marker obtained during a surgical procedure; obtain a postoperative image depicting the marker from an imaging device; determine a second pose of the marker from the postoperative image; compare the first pose and the second pose; and detect a movement of the marker based on the comparison of the first pose and the second pose, the movement detected when the second pose does not match the first pose.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a flowchart according to at least one embodiment of the present disclosure;
Fig. 3 is a flowchart according to at least one embodiment of the present disclosure;
Fig. 4 is a flowchart according to at least one embodiment of the present disclosure; and
Fig. 5 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

During a surgical procedure such as a posterior spinal procedure, a final step of the procedure may be achieving a desired alignment of the spine. Various surgical steps may be taken to support the alignment during the procedure and in most cases, surgical implants such as pedicle screws may be inserted into vertebral bodies to aid in achieving the desired alignment. One or more rods may be placed between the pedicle screws and then a surgical tool may be used to adjust rod and the pedicle screws to adjust an alignment of the spine. During such procedures, a user such as a surgeon or other medical provider may not be able to assess a result of the alignment without taking a post-operative scan using medical imaging devices such as X-Ray imaging, magnetic resonance imaging, computed tomography imaging, etc.

At least one embodiment of the present disclosure provides for connecting navigation markers or trackers to, for example, a head of a screw implanted into the vertebrae and tracking movement of the markers or trackers. The tracking may provide real-time updates of the spine on a computerized image or render which demonstrates the current position of the spine. In addition, measurements can be obtained to quantify an amount of movement of anatomical elements such as the vertebrae during alignment until the values reach calculations the surgeon intended to reach.

The navigation markers or trackers may connect rigidly to the heads or spinal screws (pedicle, cortical, etc.). The markers or trackers can be optical, magnetic, or any other type of marker. The marker or trackers can be embedded in the surgical implant (e.g., a screw, rod, etc.) or connected and disconnected to the surgical implant. The marker or tracker pose can be monitored during an alignment step of a surgical procedure. A change in the marker or tracker position can be reflected on a user interface or screen. Measurements that surgeons use to plan the spinal alignment may be calculated during the alignment according to the change in the marker or tracker pose. A notification may be generated to alert the user when the planned and/or desired alignment was reached according to the data from the marker or tracker positions. Similarly, a notification may be generated to alert the user when an unplanned movement has occurred.

This idea will (1) reduce radiation exposure to the patient and surgical staff; (2) decrease a procedure time; and (3) increase improvement of clinical outcome for patients.

Embodiments of the present disclosure provide technical solutions to one or more of the problems of (1) tracking movement of one or more anatomical elements during a surgical procedure, (2) reducing radiation exposure to a patient and surgical staff, (3) decrease a duration of a surgical procedure, and (4) increase improvements of a clinical outcome.

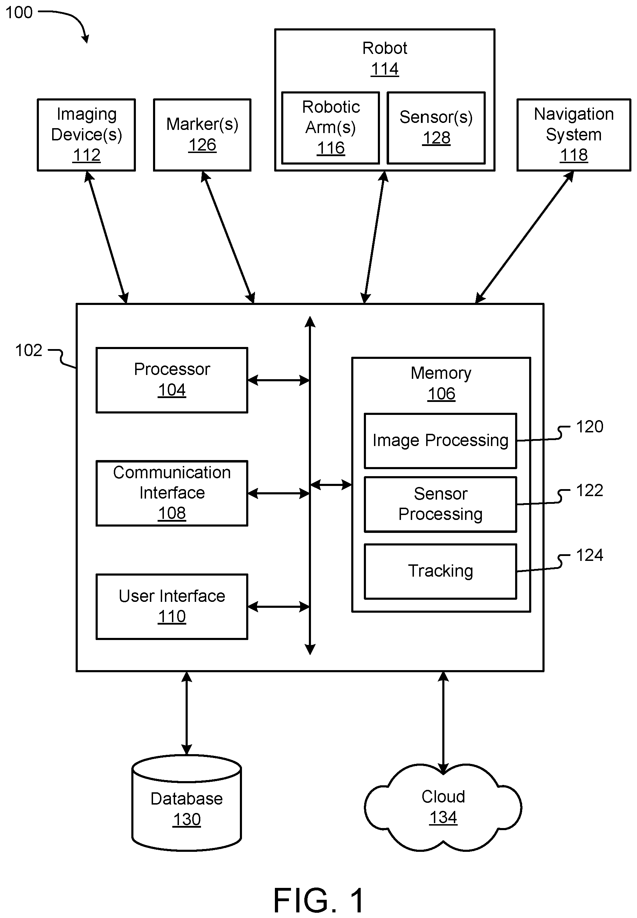

Turning first to Fig. 1, a block diagram of a system 100 according to at least one embodiment of the present disclosure is shown. The system 100 may be used to track movement of one or more anatomical elements and/or carry out one or more other aspects of one or more of the methods disclosed herein. The system 100 comprises a computing device 102, one or more imaging devices 112, a robot 114, a navigation system 118, one or more marker(s) 126, a database 130, and/or a cloud or other network 134. Systems according to other embodiments of the present disclosure may comprise more or fewer components than the system 100. For example, the system 100 may not include the imaging device 112, the robot 114, the navigation system 118, one or more components of the computing device 102, the database 130, and/or the cloud 134.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the imaging device 112, the robot 114, the navigation system 118, the database 130, and/or the cloud 134.

The memory 106 may be or comprise RAM, DRAM, SDRAM, other solid-state memory, any memory described herein, or any other tangible, non-transitory memory for storing computer-readable data and/or instructions. The memory 106 may store information or data useful for completing, for example, any step of the methods 200, 300, 400, and/or 500 described herein, or of any other methods. The memory 106 may store, for example, instructions and/or machine learning models that support one or more functions of the robot 114. For instance, the memory 106 may store content (e.g., instructions and/or machine learning models) that, when executed by the processor 104, enable image processing 120, sensor processing 122, and/or tracking 124.

The image processing 120 enables the processor 104 to process image data of an image (received from, for example, the imaging device 112, an imaging device of the navigation system 118, or any imaging device) for the purpose of, for example, identifying at least one marker of the one or more markers 126 depicting in the image. The image processing 120 may also enable the processor 104 to process the image data of the image for the purpose of, for example, determining a pose of the identified marker 126. The pose obtained from the image processing 120 may enable the navigation system 118 to determine a corresponding pose of an anatomical element to which the marker 126 is coupled to.

The sensor processing 122 enables the processor 104 to process sensor data (received from, for example, a sensor such as the sensor 128, from a marker such as the marker 126, or any sensor) for the purpose of, for example, identifying at least one marker of the one or more markers 126. For example, the marker 126 may comprise an electromagnetic sensor and/or a radio-frequency identification marker that provides sensor data that can be used to identify the marker. The sensor processing 122 may also enable the processor 104 to process the sensor data for the purpose of, for example, determining a pose of the marker 126. The pose may be determined from sensor data obtained from the marker. In other instances, the pose may be determined from sensor data obtained from the sensor 128 of the robot 114. As will be described in detail, the sensor data may be used to determine a pose of the robot 114, which may correlate to a pose of the marker 126 when the robot 114 contacts the marker 126. The pose obtained from the sensor processing 122 may enable the navigation system 118 to determine a corresponding pose of an anatomical element to which the marker 126 is coupled to or a corresponding pose of the marker 126, which may be in contact with the robot 114.

The tracking 124 enables the processor 104 (or a processor of the navigation system 118) to track the marker 126, which may be identified by the image processing 120 and/or the sensor processing 122. The tracking 124 may, for example, enable the processor 104 to compare the marker 126 at a first time period and a second time period to determine if movement of the marker 126 has occurred. In other embodiments, the tracking 124 may, for example, enable the processor 304 to compare a pose of the marker 126 (whether determined from image processing 120 and/or sensor processing 122) at a first time period and a second time period to determine a change in the pose, which indicates movement of the marker 126.

The content, if provided as in instruction, may, in some embodiments, be organized into one or more applications, modules, packages, layers, or engines. Alternatively or additionally, the memory 106 may store other types of content or data (e.g., machine learning models, artificial neural networks, deep neural networks, etc.) that can be processed by the processor 104 to carry out the various method and features described herein. Thus, although various contents of memory 106 may be described as instructions, it should be appreciated that functionality described herein can be achieved through use of instructions, algorithms, and/or machine learning models. The data, algorithms, and/or instructions may cause the processor 104 to manipulate data stored in the memory 106 and/or received from or via the imaging device 112, the robot 114, the database 130, and/or the cloud 134.

The computing device 102 may also comprise a communication interface 108. The communication interface 108 may be used for receiving image data or other information from an external source (such as the imaging device 112, the robot 114, the navigation system 118, the database 130, the cloud 134, and/or any other system or component not part of the system 100), and/or for transmitting instructions, images, or other information to an external system or device (e.g., another computing device 102, the imaging device 112, the robot 114, the navigation system 118, the database 130, the cloud 134, and/or any other system or component not part of the system 100). The communication interface 108 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, screen, touchscreen, and/or any other device for receiving information from a user and/or for providing information to a user. The user interface 110 may be used, for example, to receive a user selection or other user input regarding any step of any method described herein. Notwithstanding the foregoing, any required input for any step of any method described herein may be generated automatically by the system 100 (e.g., by the processor 104 or another component of the system 100) or received by the system 100 from a source external to the system 100. In some embodiments, the user interface 110 may be useful to allow a surgeon or other user to modify instructions to be executed by the processor 104 according to one or more embodiments of the present disclosure, and/or to modify or adjust a setting of other information displayed on the user interface 110 or corresponding thereto.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computer device 102.

The imaging device 112 may be operable to image anatomical feature(s) (e.g., a bone, veins, tissue, etc.) and/or other aspects of patient anatomy to yield image data (e.g., image data depicting or corresponding to a bone, veins, tissue, etc.). "Image data" as used herein refers to the data generated or captured by an imaging device 112, including in a machine-readable form, a graphical/visual form, and in any other form. In various examples, the image data may comprise data corresponding to an anatomical feature of a patient, or to a portion thereof. The image data may be or comprise a preoperative image, an intraoperative image, a postoperative image, or an image taken independently of any surgical procedure. In some embodiments, a first imaging device 112 may be used to obtain first image data (e.g., a first image) at a first time, and a second imaging device 112 may be used to obtain second image data (e.g., a second image) at a second time after the first time. The imaging device 112 may be capable of taking a 2D image or a 3D image to yield the image data. The imaging device 112 may be or comprise, for example, an ultrasound scanner (which may comprise, for example, a physically separate transducer and receiver, or a single ultrasound transceiver), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography (OCT) scanner, an endoscope, a microscope, an optical camera, a thermographic camera (e.g., an infrared camera), a radar system (which may comprise, for example, a transmitter, a receiver, a processor, and one or more antennae), or any other imaging device 112 suitable for obtaining images of an anatomical feature of a patient. The imaging device 112 may be contained entirely within a single housing, or may comprise a transmitter/emitter and a receiver/detector that are in separate housings or are otherwise physically separated.

In some embodiments, the imaging device 112 may comprise more than one imaging device 112. For example, a first imaging device may provide first image data and/or a first image, and a second imaging device may provide second image data and/or a second image. In still other embodiments, the same imaging device may be used to provide both the first image data and the second image data, and/or any other image data described herein. The imaging device 112 may be operable to generate a stream of image data. For example, the imaging device 112 may be configured to operate with an open shutter, or with a shutter that continuously alternates between open and shut so as to capture successive images. For purposes of the present disclosure, unless specified otherwise, image data may be considered to be continuous and/or provided as an image data stream if the image data represents two or more frames per second.

The robot 114 may be any surgical robot or surgical robotic system. The robot 114 may be or comprise, for example, the Mazor X^{™} Stealth Edition robotic guidance system. The robot 114 may be configured to position the imaging device 112 at one or more precise position(s) and orientation(s), and/or to return the imaging device 112 to the same position(s) and orientation(s) at a later point in time. The robot 114 may additionally or alternatively be configured to manipulate a surgical tool (whether based on guidance from the navigation system 118 or not) to accomplish or to assist with a surgical task. In some embodiments, the robot 114 may be configured to hold and/or manipulate an anatomical element during or in connection with a surgical procedure. The robot 114 may comprise one or more robotic arms 116. In some embodiments, the robotic arm 116 may comprise a first robotic arm and a second robotic arm, though the robot 114 may comprise more than two robotic arms. In some embodiments, one or more of the robotic arms 116 may be used to hold and/or maneuver the imaging device 112. In embodiments where the imaging device 112 comprises two or more physically separate components (e.g., a transmitter and receiver), one robotic arm 116 may hold one such component, and another robotic arm 116 may hold another such component. Each robotic arm 116 may be positionable independently of the other robotic arm. The robotic arms 116 may be controlled in a single, shared coordinate space, or in separate coordinate spaces.

The robot 114, together with the robotic arm 116, may have, for example, one, two, three, four, five, six, seven, or more degrees of freedom. Further, the robotic arm 116 may be positioned or positionable in any pose, plane, and/or focal point. The pose includes a position and an orientation. As a result, an imaging device 112, surgical tool, or other object held by the robot 114 (or, more specifically, by the robotic arm 116) may be precisely positionable in one or more needed and specific positions and orientations.

The robot 114 may comprise one or more sensors 128. The sensor 128 may be a position sensor, a proximity sensor, a magnetometer, or an accelerometer. In some embodiments, the sensor 128 may be a linear encoder, a rotary encoder, or an incremental encoder. Other types of sensors may also be used as the sensor 128. The one or more sensors 128 may be positioned, for example, on the robotic arm 116 or elsewhere. Data from the sensor(s) 128 may be provided to a processor of the robot 114, to the processor 104 of the computing device 102, and/or to the navigation system 118. The data may be used to calculate a position and/or orientation in space of the robotic arm 116 relative to one or more coordinate systems. The calculation may be based not just on data received from the sensor(s) 128, but also on data or information (such as, for example, physical dimensions) about, for example, the robot 114 or a portion thereof, or any other relevant object, which data or information may be stored, for example, in a memory 116 of a computing device 102 or in any other memory.

The system 100 may comprise the markers 126. In some embodiments, the markers 126 may be placed on an anatomical element, a surgical implant, a surgical instrument, a surgical tool, the robot 114 (including, e.g., on the robotic arm 116), the imaging device 112, or any other object in the surgical space. In some embodiments, the markers 126 may be oriented by the robot 114. In other embodiments, the markers 126 may be oriented manually by, for example, a user such as a surgeon or other medical personnel. The markers 126 may be tracked by the navigation system 118, and the results of the tracking may be used by the robot 114 and/or by an operator of the system 100 or any component thereof. In some embodiments, the navigation system 118 can be used to track other components of the system (e.g., imaging device 112) and the system can operate without the use of the robot 114 (e.g., with the surgeon manually manipulating the imaging device 112 and/or one or more surgical tools, based on information and/or instructions generated by the navigation system 118, for example).

The marker 126 may comprise one or more active markers, one or more passive markers, or a combination of active and passive markers. The marker 126 may be, for example, a magnetic marker, an optical marker, light emitting diodes, infrared light emitting diodes, electromagnetic trackers, radio-frequency identification trackers, reflective markers, or the like. In some embodiments, the marker 126 may be coupled to an anatomical element via a surgical implant. For example, the surgical implant may be a pedicle screw and the anatomical element may be a vertebrae. In such examples, the marker 126 may be coupled to the pedicle screw, which may be screwed into the vertebrae. In some embodiments, the marker 126 may be embedded onto any portion of the pedicle screw (e.g., the pedicle screw, a closure of the pedicle screw, and/or a head or receiver of the pedicle screw). In other embodiments, the marker 126 may be releasably coupled to the pedicle screw. For example, the marker 126 may comprise a threaded protrusion and the screw head may comprise a threaded cavity (e.g., a head or receiver) configured to receive the threaded protrusion. In other words, the marker 126 may be screwed into or unscrewed from the head or receiver of the pedicel screw. It will be appreciated that the marker 126 may be coupled to any surgical implant (e.g., rod, screws, plates, etc.) and/or any anatomical element. For example, the marker 126 may be clamped to or otherwise attached to a rod. It will also be appreciated that the marker 126 may be coupled to the anatomical element without the surgical implant (e.g., the marker 126 may be directly coupled to the anatomical element).

The navigation system 118 may provide navigation for a surgeon and/or a surgical robot during an operation. The navigation system 118 may be any now-known or future-developed navigation system, including, for example, the Medtronic StealthStation^{™} S8 surgical navigation system or any successor thereof. The navigation system 118 may include one or more cameras or other sensor(s) for tracking one or more reference markers, navigated trackers, or other objects within the operating room or other room in which some or all of the system 100 is located. The one or more cameras may be optical cameras, infrared cameras, or other cameras. In some embodiments, the navigation system 118 may comprise one or more electromagnetic sensors. In various embodiments, the navigation system 118 may be used to track a position and orientation (e.g., a pose) of the imaging device 112, the robot 114 and/or robotic arm 116, the markers 126, and/or one or more surgical tools (or, more particularly, to track a pose of a navigated tracker attached, directly or indirectly, in fixed relation to the one or more of the foregoing). The navigation system 118 may include a display for displaying one or more images from an external source (e.g., the computing device 102, imaging device 112, or other source) or for displaying an image and/or video stream from the one or more cameras or other sensors of the navigation system 118. In some embodiments, the system 100 can operate without the use of the navigation system 118. The navigation system 118 may be configured to provide guidance to a surgeon or other user of the system 100 or a component thereof, to the robot 114, or to any other element of the system 100 regarding, for example, a pose of one or more anatomical elements, whether or not a tool is in the proper trajectory, and/or how to move a tool into the proper trajectory to carry out a surgical task according to a preoperative or other surgical plan.

The database 130 may store information that correlates one coordinate system to another (e.g., one or more robotic coordinate systems to a patient coordinate system and/or to a navigation coordinate system). The database 130 may additionally or alternatively store, for example, one or more surgical plans (including, for example, pose information about a target and/or image information about a patient's anatomy at and/or proximate the surgical site, for use by the robot 114, the navigation system 118, and/or a user of the computing device 102 or of the system 100); one or more images useful in connection with a surgery to be completed by or with the assistance of one or more other components of the system 100; and/or any other useful information. The database 130 may be configured to provide any such information to the computing device 102 or to any other device of the system 100 or external to the system 100, whether directly or via the cloud 134. In some embodiments, the database 130 may be or comprise part of a hospital image storage system, such as a picture archiving and communication system (PACS), a health information system (HIS), and/or another system for collecting, storing, managing, and/or transmitting electronic medical records including image data.

The cloud 134 may be or represent the Internet or any other wide area network. The computing device 102 may be connected to the cloud 134 via the communication interface 108, using a wired connection, a wireless connection, or both. In some embodiments, the computing device 102 may communicate with the database 130 and/or an external device (e.g., a computing device) via the cloud 134.

The system 100 or similar systems may be used, for example, to carry out one or more aspects of any of the methods 200, 300, 400, and/or 500 described herein. The system 100 or similar systems may also be used for other purposes.

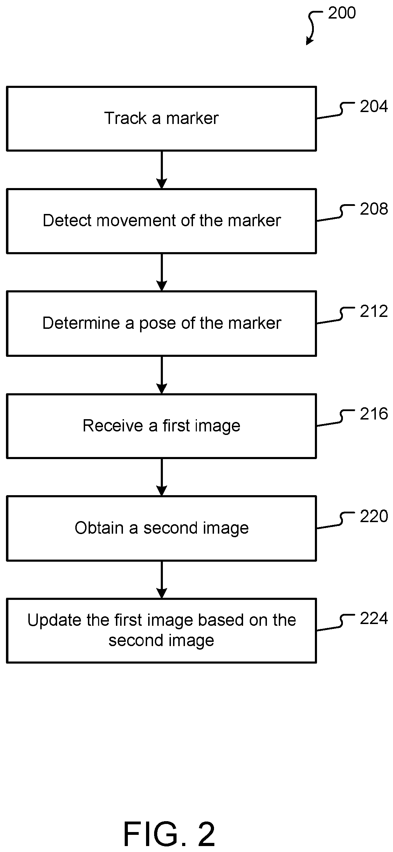

Fig. 2 depicts a method 200 that may be used, for example, for tracking movement of an anatomical element.

The method 200 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 200. The at least one processor may perform the method 200 by executing elements stored in a memory such as the memory 106. The elements stored in the memory and executed by the processor may cause the processor to execute one or more steps of a function as shown in method 200. One or more portions of a method 200 may be performed by the processor executing any of the contents of memory, such as an image processing 120, sensor processing 122, and/or a tracking 124.

The method 200 comprises tracking a marker (step 204). The marker may be the same as or similar to the marker 126. The marker may be coupled to an anatomical element. In some embodiments, the marker may be coupled to the anatomical element via a surgical implant. For example, the surgical implant may be a pedicle screw and the anatomical element may be a vertebrae. In such examples, the marker may be coupled to the pedicle screw, which may be screwed into the vertebrae. In some embodiments, the marker may be embedded onto any portion of the pedicle screw (e.g., the pedicle screw, a closure of the pedicle screw, and/or a head or receiver of the pedicle screw). In other embodiments, the marker may be releasably coupled to the pedicle screw. For example, the marker may comprise a threaded protrusion and the screw head may comprise a threaded cavity (e.g., a head or receiver) configured to receive the threaded protrusion. In other words, the marker may be screwed into or unscrewed from the head or receiver of the pedicel screw.

Tracking the marker may comprise a navigation system such as the navigation system 118 tracking the marker. In some embodiments, a processor such as the processor 104 or a processor of the navigation system may use an image processing such as the image processing 120 to process image data and/or a sensor processing such as the sensor processing 122 to process sensor data to identify a marker for tracking. The image processing enables the processor to process image data of an image received from, for example, an imaging device such as the imaging device 112, an imaging device of the navigation system, or any other imaging device for the purpose of identifying one or more markers depicted in the image. Similarly, the sensor processing enables the processor to process sensor data from, for example, a sensor such as the sensor 128 and/or sensor data correlating to the marker for the purpose of identifying the marker.

Tracking the marker may comprise the processor using a tracking such as the tracking 124 to track the marker identified by the imaging processing and/or the sensor processing. The tracking may, for example, enable the processor to compare the identified marker at a first time period and a second time period to determine if movement of the identified marker has occurred between the first time period and the second time period. In other embodiments, the tracking may, for example, enable the processor to compare a pose of the identified marker (whether determined from image processing and/or sensor processing) at a first time period and a second time period to determine a change in the pose (which may indicate movement of the identified marker).

In some embodiments, such as a spinal alignment procedure, movement of an anatomical element such as, for example, a vertebrae may be tracked to determine when a pose of the anatomical element (as tracked by, for example, the navigation system) has reached a desired pose. In other embodiments, movement of the anatomical element may not be desired and thus, the marker may be tracked to monitor the marker for undesired movement.

The method 200 also comprises detecting movement of the marker (step 208). During a surgical procedure, the marker may be tracked for movement - whether due to movement of the patient, an operating table, or the surgical procedure (e.g., movement of the spine). Movement of the marker may indicate that the anatomical element has moved.

In some embodiments, a navigation system such as the navigation system 118 may be used to detect the movement of the marker. In such embodiments, the navigation system may detect the movement of the marker by, for example, tracking a pose of the marker (whether by using imaging, sensors, or the like). More specifically, detecting the movement may comprise comparing a first pose of the marker at a first time period and a second pose of the marker at a second time period. The first pose and the second post may be obtained from, for example, the navigation system 118. The first pose may be compared to the second pose and a difference in the first pose and the second pose may indicate movement of the marker. It will be appreciated that subsequent poses may be continuously tracked and obtained from, for example, the navigation system. It will be appreciated that in some embodiments the first pose and the second pose may be obtained from the image processing or the sensor processing described in step 204.

In other embodiments, detecting movement of the marker may be based on a comparison of a first image and a second image, which may each be received from an imaging device such as the imaging device 112 (or an imaging device of, for example, the navigation system, or any other imaging device). Each of the first image and the second image may depict the marker. The second image may be obtained at a time period after the first image. The first image may be obtained preoperatively or intraoperatively. In some embodiments, detecting the movement of the marker may comprise superimposing the second image over the first image and comparing differences between the marker depicted in the first image and the second image. The differences may be determined by visually detecting the differences of the marker between the first image and the second image. In other instances, the differences may be determined automatically by, for example, the processor. For example, the processor may compare each pixel of the first image to each corresponding pixel of the second image and differences in pixels may indicate a difference between the first image and the second image.

It will be appreciated that in some embodiments, the second image may be updated using consecutive second images to determine how much change to a marker has been imparted to an anatomical element (such as, for example, a spine) intraoperatively. In other words, multiple second images may be obtained and subsequent second images may be compared to former second images to determine a change in the markers (and thus, the anatomical element to which the markers are attached to) between the former and subsequent second images. The detected movement or change (whether determined by using the navigation system, the imaging processing, and/or the sensor processing) can be compared to the first image, which may depict a pre-operative shape of the anatomical element. In such embodiments, the movement or changes detected can be used to assess, for example, a global alignment of a patient's spine and compensatory mechanisms.

The method 200 also comprises determining a pose of the marker (step 212). The pose of the marker may be determined by, for example, a navigation system such as the navigation system 118. In some embodiments, determining a pose of the marker may be based on the movement detected in the step 208 described above. In embodiments where detecting the movement is not based on the pose of the marker - such as where a first image and a second image are superimposed on each other or compared to each other to detect the movement - the pose of the marker may be determined when the movement has been detected. The pose may be determined by, for example, the processor using the image processing to process the second image to obtain a pose of the marker from the second image. The pose may also be determined by the processor using the sensor processing to process sensor data received from, for example, a sensor such as the sensor 126 of a robotic arm such as the robotic arm 116. More specifically, the robotic arm may contact the marker and the sensor data may be processed using the sensor processing to obtain a pose of the robotic arm as the robotic arm is contacting the marker. Thus, the pose of the robotic arm may correlate to a pose of the marker. In some embodiments where the marker comprises an electromagnetic tracker and/or a radio-frequency identification tracker, sensor data from the marker may be processed by the processor using the sensor processing to obtain the pose of the marker.

The method 200 also comprises receiving a first image (step 216). In some embodiments, the first image may be the same as or similar to the first image described above in steps 208 and/or 212. In other embodiments, the first image may be a preoperative image or an image obtained prior to a start of a surgical procedure. The first image may be received via a user interface such as the user interface 110 and/or a communication interface such as the communication interface 108 of a computing device such as the computing device 102, and may be stored in a memory such as the memory 106 of the computing device. The first image may also be received from an external database or image repository (e.g., a hospital image storage system, such as a picture archiving and communication system (PACS), a health information system (HIS), and/or another system for collecting, storing, managing, and/or transmitting electronic medical records including image data), and/or via the Internet or another network. In other embodiments, the first image may be received or obtained from an imaging device such as the imaging device 112, which may be any imaging device such as an MRI scanner, a CT scanner, any other X-ray based imaging device, or an ultrasound imaging device. The first image may also be generated by and/or uploaded to any other component of a system such as the system 100. **In** some embodiments, the first image may be indirectly received via any other component of the system or a node of a network to which the system is connected.

The first image may be a 2D image or a 3D image or a set of 2D and/or 3D images. The first image may depict a patient's anatomy or portion thereof. **In** some embodiments, the first image may be stored in a system (e.g., a system 100) and/or one or more components thereof (e.g., a database 130). The stored first image may then be received (e.g., by a processor 104), as described above, preoperatively (e.g., before the surgery) and/or intraoperatively (e.g., during surgery). **In** some embodiments, the first image may depict multiple anatomical elements associated with the patient anatomy, including incidental anatomical elements (e.g., ribs or other anatomical objects on which a surgery or surgical procedure will not be performed) in addition to target anatomical elements (e.g., vertebrae or other anatomical objects on which a surgery or surgical procedure is to be performed). The first image may comprise various features corresponding to the patient's anatomy and/or anatomical elements (and/or portions thereof), including gradients corresponding to boundaries and/or contours of the various depicted anatomical elements, varying levels of intensity corresponding to varying surface textures of the various depicted anatomical elements, combinations thereof, and/or the like. The first image may depict any portion or part of patient anatomy and may include, but is in no way limited to, one or more vertebrae, ribs, lungs, soft tissues (e.g., skin, tendons, muscle fiber, etc.), a patella, a clavicle, a scapula, combinations thereof, and/or the like.

The method 200 also comprises obtaining a second image (step 220). The second image may be obtained from an imaging device such as the imaging device 112 and may depict the marker. In some embodiments, the first image may be obtained preoperatively and the second image may be obtained intraoperatively. In other embodiments, the first image and the second image may both be obtained intraoperatively. In at least one embodiment, the first image may be obtained using a first imaging modality and the second image may be obtained using a second imaging modality. For example, the first image may be obtained using X-ray imaging and the second image may be obtained using imaging free of ionizing radiation (e.g., optical, ultrasound, etc.).

The method 200 also comprises updating the first image based on the second image (step 224). In some embodiments, the first image may be updated based on the pose of the marker as determined from the second image (by, for example, the processor using the image processing to obtain the pose from image data of the second image) after movement have been detected, as determined in, for example step 212. In other embodiments, the first image may be updated based on the pose of the marker as obtained from, for example, the step 204. Updating the first image may comprise updating a pose of the anatomical element. In embodiments where the first image may comprise a three-dimensional representation of the anatomical element, a pose of the three-dimensional representation of the anatomical element may be updated. In such embodiments, a boundary comprising a surface mesh of the anatomical element may be updated based on the determined pose.

In some instances, the step 224 may not occur if movement is not detected in, for example, the step 208. In other instances, the step 224 may occur regardless of detected movement.

The present disclosure encompasses embodiments of the method 200 that comprise more or fewer steps than those described above, and/or one or more steps that are different than the steps described above.

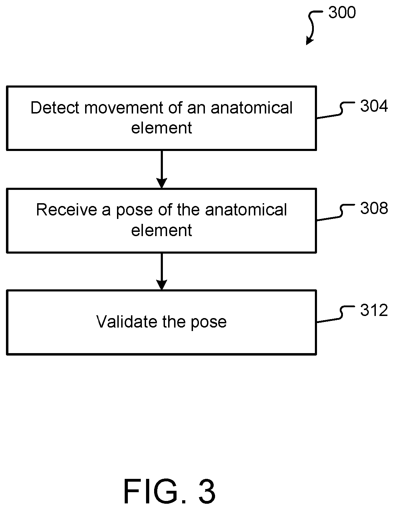

Fig. 3 depicts a method 300 that may be used, for example, for validating a pose of an anatomical element.

The method 300 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 300. The at least one processor may perform the method 300 by executing elements stored in a memory such as the memory 106. The elements stored in memory and executed by the processor may cause the processor to execute one or more steps of a function as shown in method 300. One or more portions of a method 300 may be performed by the processor executing any of the contents of memory, such as an image processing 120, sensor processing 122, and/or a tracking 124.

The method 300 comprises detecting movement of an anatomical element (step 304). The step 304 may be the same as or similar to the step 208 of the method 200 described above. It will be appreciated that in some embodiments, a marker such as the marker 126 may be coupled to the anatomical element and movement of the marker may correlate to movement of the anatomical element. Thus, the step 304 may comprise detecting movement of a marker coupled to the anatomical element and the step 304 may also comprise correlating the movement of the marker to movement of the anatomical element.

The movement of the anatomical element may be detected after a surgical procedure or step has been completed. For example, the movement of the anatomical element may be detected or measured after a surgical step has been completed that may result in movement of a patient's spine. In such examples, movement of one or more vertebrae may be detected or measured.

The method 300 also comprises determining a pose of the anatomical element (step 308). The step 308 may be the same as or similar to the step 212 of the method 200 described above. Similarly to the step 304, it will be appreciated that in some embodiments, the marker may be coupled to the anatomical element. Thus, the step 308 may comprise determining the pose of the marker coupled to the anatomical element and the step 308 may also comprise correlating the pose of the marker to the pose of the anatomical element.

The method 300 also comprises validating the pose (step 312). The pose of the anatomical element may be validated by comparing the pose as determined in, for example, the step 308 above, to a predetermined pose of, for example, a surgical plan. The predetermined pose may be, for example, determined automatically using artificial intelligence and training data (e.g., historical cases) in some embodiments. The historical cases may be, for example, historical outcomes of surgical procedures performed on historical patients. In other embodiments, the predetermined pose may be or comprise, or be based on, surgeon input received via the user interface. In further embodiments, the predetermined pose may be determined automatically using artificial intelligence, and may thereafter be reviewed and approved (or modified) by a surgeon or other user.

Validating the pose of the anatomical element may provide confirmation that a desired outcome of a surgical procedure has been achieved. For example, a surgical procedure may comprise adjusting an alignment of a patient's spine using spinal screws and/or rods and the outcome may comprise movement of the patient's spine, and more specifically, movement of one or more vertebrae. In such embodiments, an actual pose of one or more vertebrae (as determined in, for example, the step 308 above) may be compared to a corresponding one or more predetermined poses after the surgical procedure has been completed. The comparison may be used to determine if the one or more vertebrae have reached the desired one or more predetermined poses. Each of the one or more vertebrae may be validated when a pose of the vertebra substantially matches a corresponding predetermined pose. Each of the one or more vertebrae may also be validated when a pose of the anatomical element is within a range (such as a position threshold) of a predetermined pose.

Such validation may occur in real-time during the surgical procedure and may provide valuable information to a user such as a surgeon or other medical personnel. For example, the validation may confirm to the user that a desired alignment of the spine has been achieved. On the contrary, a lack of validation may notify the user that the desired alignment of the spine has not been achieved. Further, in embodiments where the anatomical element is tracked by a navigation system such as the navigation system 118, the anatomical element may be tracked without the use of imaging using ionizing radiation (e.g., X-rays). It will be appreciated that in some embodiments, the anatomical element may be tracking using imaging free of ionizing radiation.

It will be appreciated that the steps 304 and 308 may be continuously repeated until the pose of each of one or more anatomical elements as determined in the step 308 is validated in the step 312. In other words, the pose of the anatomical element may be continuously determined and/or tracked until the pose substantially matches a corresponding predetermined pose.

The present disclosure encompasses embodiments of the method 300 that comprise more or fewer steps than those described above, and/or one or more steps that are different than the steps described above.

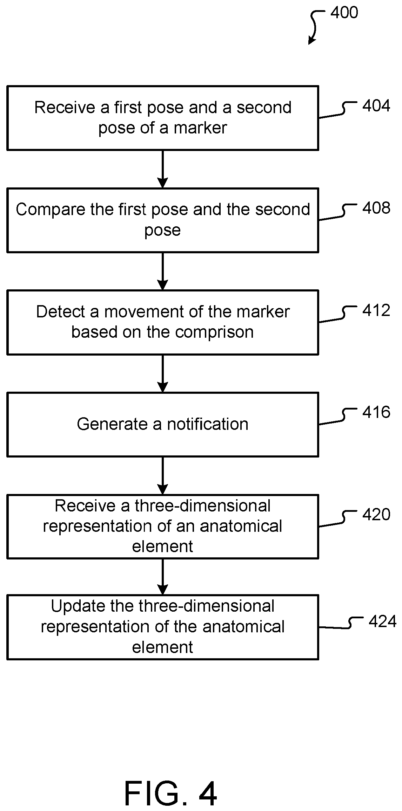

Fig. 4 depicts a method 400 that may be used, for example, for tracking movement of an anatomical element and updating a three-dimensional representation of an anatomical element.

The method 400 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 400. The at least one processor may perform the method 400 by executing elements stored in a memory such as the memory 106. The elements stored in memory and executed by the processor may cause the processor to execute one or more steps of a function as shown in method 400. One or more portions of a method 400 may be performed by the processor executing any of the contents of memory, such as an image processing 120, sensor processing 122, and/or a tracking 124.

The method 400 comprises receiving a first pose and a second pose of a marker (step 404). The marker may be the same as or similar to the marker 126 and may be coupled to an anatomical element. The first pose may be received at a first time period and the second pose may be received at a second time period. The second time period may be after the first time period. The first pose and the second pose may be, in some embodiments, received from a navigation system such as the navigation system 118. In other embodiments, the first pose and the second pose may be determined by, for example, a processor such as the processor 104 using image processing such as the image processing 120 to process a first image and a second image (which may each depict the marker) to obtain a pose of the marker from the first image and the second image. In some embodiments where the marker comprises an electromagnetic tracker and/or a radio-frequency identification tracker, sensor data from the marker may be processed by the processor (which may be a processor of the navigation system) using a sensor processing such as the sensor processing 122 to obtain the first pose and the second pose of the marker.

The first pose and the second pose may also be determined by the processor using sensor processing to process sensor data received from, for example, a sensor such as the sensor 126 of a robotic arm such as the robotic arm 116. More specifically, the robotic arm may contact the marker at a first time period and a second time period and the sensor data may be processed using the sensor processing to obtain a first pose at the first time period and a second pose at the second time period of the robotic arm as the robotic arm is contacting the marker. Thus, the first pose and the second pose of the robotic arm may correlate to a first pose and a second pose of the marker at the first time period and the second time period, respectively.

The method 400 also comprises comparing the first pose and the second pose (step 408). The first pose and the second pose may be compared to determine if movement of the marker has occurred. If the first pose and the second pose do not match (e.g., a position and/or an orientation of the first pose and the second pose do not match), then this indicates that movement has occurred. For example, an X-coordinate of the first pose and the second pose may not match. If the first pose and the second pose match, then this indicates that movement has not occurred.

The method 400 also comprises detecting a movement of the marker based on the comparison (step 412). The step may be the same as or similar to the step 208 of the method 200 described above.

The method 400 also comprises generating a notification (step 416). The notification may be a visual notification, an audible notification, or any type of notification communicated to a user. The notification may be communicated to the user via a user interface such as the user interface 110. In some embodiments, the notification may be automatically generated by the processor 104. In other embodiments, the notification may be automatically generated by any component of a system such as the system 100.

In some embodiments, the notification is based on a desired pose. The desired pose may be received from, for example, a surgical plan. In some embodiments, movement of the marker (which correlates to movement of the anatomical element) may be desired. For example, during a spinal alignment, one or more vertebrae of a patient are moved to move the spine into a desired alignment. Thus, the notification may be based on the desired pose. The notification may notify to a user that a pose of the anatomical element has reached the desired pose or that the anatomical element has not reached the desired pose. The notification may also notify to the user that the pose of the anatomical element is within a range (such as a position threshold) of the desired pose. For example, the notification may alert the user that the anatomical element is within 10mm of the desired pose.

In other embodiments, the notification may be based on a predetermined movement threshold for movement of an anatomical element. The predetermined movement threshold may correlate to a maximum allowable movement of the anatomical element and the notification may be generated when the movement meets or exceeds the corresponding predetermined movement threshold. The predetermined movement threshold may be determined automatically using artificial intelligence and training data (e.g., historical cases) in some embodiments. In other embodiments, the predetermined movement threshold may be or comprise, or be based on, surgeon input received via the user interface. In further embodiments, the predetermined movement threshold may be determined automatically using artificial intelligence, and may thereafter be reviewed and approved (or modified) by a surgeon or other user.

The method 400 also comprises receiving a three-dimensional representation of an anatomical element (step 420). The three-dimensional representation may depict one or more objects and/or anatomical elements. In embodiments where the three-dimensional representation depicts at least one anatomical element, the three-dimensional representation may comprise hard tissue information and/or soft tissue information.

The method 400 also comprises updating the three-dimensional representation of the anatomical element (step 424). In some embodiments, at least a portion of the three-dimensional representation may be updated based on the detected movement. In some embodiments, the updated portion may be updated based on a pose of the marker (and the corresponding anatomical element) after the movement has been detected. In some embodiments, the pose may comprise the second pose received in step 404. In other embodiments, the pose may be determined after the movement is detected such as, for example, described in step 212 of the method 200 above. In some embodiments, the entire three-dimensional representation may be updated. For example, movement of a vertebra may cause an adjacent vertebra to move. In such example, a three-dimensional representation of an entire spinal region may be updated to reflect movement of the initial anatomical element and anatomical elements affected by movement of the initial anatomical element.

In some instances, the step 424 may not occur if movement is not detected in, for example, the step 412. In other instances, the step 424 may occur regardless of detected movement. In still other embodiments, the step 424 may occur if a movement threshold, such as the movement threshold described with respect to step 416 is met or exceeded by the detected movement.

The present disclosure encompasses embodiments of the method 400 that comprise more or fewer steps than those described above, and/or one or more steps that are different than the steps described above.

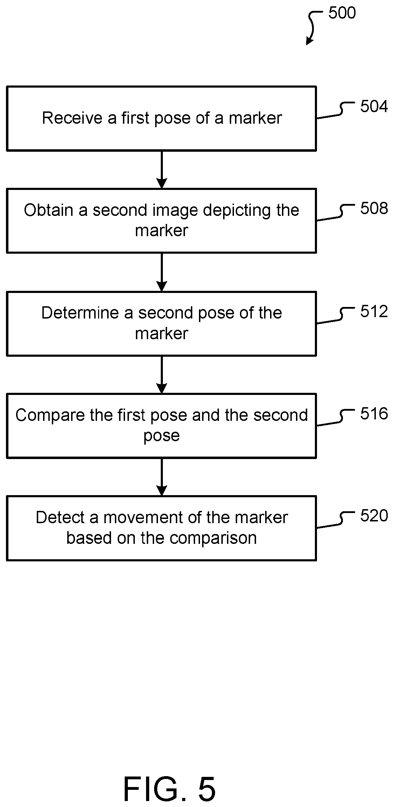

Fig. 5 depicts a method 500 that may be used, for example, for tracking movement of an anatomical element.

The method 500 (and/or one or more steps thereof) may be carried out or otherwise performed, for example, by at least one processor. The at least one processor may be the same as or similar to the processor(s) 105 of the computing device 102 described above. The at least one processor may be part of a robot (such as a robot 114) or part of a navigation system (such as a navigation system 118). A processor other than any processor described herein may also be used to execute the method 500. The at least one processor may perform the method 500 by executing elements stored in a memory such as the memory 106. The elements stored in memory and executed by the processor may cause the processor to execute one or more steps of a function as shown in method 500. One or more portions of a method 500 may be performed by the processor executing any of the contents of memory, such as an image processing 120, sensor processing 122, and/or a tracking 124.

The method 500 comprises receiving a first pose of a marker (step 504). The step 504 may be the same as or similar to the step 404 of method 400 described above. The marker may be the same as or similar to the marker 126. The marker may be coupled to the anatomical element. As previously described, the marker may be coupled to the anatomical element via a surgical implant. In some embodiments, the first pose of the marker is obtained intraoperatively and, in some instances, the first pose of the marker is obtained at an end of a surgical operation. The first pose may be stored in a database such as the database 130 for later retrieval.

The method 500 also comprises obtaining a second image depicting the marker (step 508). The step 508 may be the same as or similar to the step 220 of the method 400 described above. More specifically, the second image may be a postoperative image. In such instances, the marker may comprise a marker configured to remain in a patient and is configured to be tracked within the patient. For example, the marker may be an electromagnetic tracker or a radio-frequency identification tracker. In such examples, the marker may be integrated with a surgical implant that was implanted into the patient during the surgical operation.

The method 500 also comprises determining a second pose of the marker (step 512). The step 512 may be the same as or similar to the step 212 of the method 200 described above.

The method 500 also comprises comparing the first pose and the second pose (step 516). The step 516 may be the same as or similar to the step 408 of the method 400 described above.

The method 500 also comprises detecting a movement of the marker based on the comparison (step 520). The step 520 may be the same as or similar to the step 412 of the method 400 above. In some embodiments, movement of the marker may indicate that movement of the corresponding anatomical element to which the marker is coupled to has moved after completion of a surgical operation. More specifically, because the first pose of the marker correlates to a first pose of the anatomical element intraoperatively (and in some instances, at an end of a surgical operation) and the second pose of the marker correlates to a second pose of the anatomical element postoperatively, the detected movement correlates to a movement of the anatomical element after the surgery has been completed. For example, the second pose may be obtained at a follow-up examination or visit with a surgical provider such as, for example, a surgeon. The detected movement (or lack thereof) may be useful to a surgical provider to determine an outcome of the surgical operation.

The present disclosure encompasses embodiments of the method 500 that comprise more or fewer steps than those described above, and/or one or more steps that are different than the steps described above.

As noted above, the present disclosure encompasses methods with fewer than all of the steps identified in Figs. 2, 3, 4, and 5 (and the corresponding description of the methods 200, 300, 400, and 500), as well as methods that include additional steps beyond those identified in Figs. 2, 3, 4, and 5 (and the corresponding description of the methods 200, 300, 400, and 500). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure.

## Claims

1. A system (100) for tracking movement of an anatomical element, the system comprising:
a marker (126) coupled to the anatomical element;
a processor (104); and
a memory (106) storing data for processing by the processor (104), the data, when processed, causes the processor (104) to:
track the marker (126);
detect a movement of the marker (126);
determine a pose of the marker (126) based on the movement; and
receive a first image of the anatomical element, the first image obtained preoperatively,
**characterized in that**
the memory (106) stores further data for processing by the processor (104) that, when processed, causes the processor (104) to:
obtain a second image from an imaging device (112), the second image depicting the marker (126) and the anatomical element; and
update the first image of the anatomical element based on the second image (126) of the anatomical element and the detected movement.

2. The system (100) of claim 1, wherein the marker (126) comprises at least one of an optical marker (126), an electromagnetic tracker, a radio-frequency identification tracker, a magnetic marker, a light emitting diode, or an infrared light emitting diode.

3. The system (100) of claim 1, wherein the marker (126) is integrated with a surgical implant, the surgical implant attached to the anatomical element.

4. The system (100) of claim 3, wherein the surgical implant is a rod.

5. The system (100) of claim 1, wherein the anatomical element is a vertebra and the marker (126) is releasably secured to a screw head of a screw embedded in the vertebra.

6. The system (100) of claim 5, wherein the screw head comprises a threaded cavity configured to receive a threaded protrusion of the marker (126).

7. The system (100) of claim 6, wherein the memory (106) stores further data for processing by the processor (104) that, when processed, causes the processor (104) to:
compare the pose of the marker (126) to a predetermined pose; and
validate the pose of the marker (126) when the pose substantially matches the predetermined pose.

8. The system (100) of claim 1, wherein the pose comprises at least one of a first pose of the marker (126) and a second pose of the marker (126).

9. The system (100) of claim 8, wherein detecting movement of the marker (126) comprises comparing the first pose of the marker (126) and the second pose of the marker (126).

10. The system (100) of claim 1, wherein the pose is obtained from at least one of a navigation system (118) configured to track a pose of the marker (126) or a robotic arm (116) orienting the marker (126), the robotic arm (116) comprising at least one sensor (128) for sensing a pose of the robotic arm (116).

## Patentansprüche

1. System (100) zur Verfolgung der Bewegung eines anatomischen Elements, wobei das System umfasst:
einen Marker (126), der an das anatomische Element gekoppelt ist;
einen Prozessor (104); und
einen Speicher (106), der Daten zum Verarbeiten durch den Prozessor (104) speichert, wobei die Daten, wenn sie verarbeitet werden, den Prozessor (104) veranlassen zum:
Verfolgen des Markers (126);
Erfassen einer Bewegung des Markers (126);
Bestimmen einer Pose des Markers (126) basierend auf der Bewegung; und
Empfangen eines ersten Bildes des anatomischen Elements, wobei das erste Bild präoperativ erhalten wird,
**dadurch gekennzeichnet, dass**
der Speicher (106) weitere Daten zum Verarbeiten durch den Prozessor (104) speichert, die, wenn sie verarbeitet werden, den Prozessor (104) veranlassen zum:
Erhalten eines zweiten Bildes von einer Bildgebungsvorrichtung (112), wobei das zweite Bild den Marker (126) und das anatomische Element darstellt; und
Aktualisieren des ersten Bildes des anatomischen Elements basierend auf dem zweiten Bild (126) des anatomischen Elements und der erfassten Bewegung.

2. System (100) nach Anspruch 1, wobei der Marker (126) mindestens eines von einem optischen Marker (126), einem elektromagnetischen Tracker, einem Radiofrequenz-Identifikations-Tracker, einem magnetischen Marker, einer Leuchtdiode oder einer Infrarot-Leuchtdiode umfasst.

3. System (100) nach Anspruch 1, wobei der Marker (126) mit einem chirurgischen Implantat integriert ist, wobei das chirurgische Implantat an dem anatomischen Element befestigt ist.

4. System (100) nach Anspruch 3, wobei das chirurgische Implantat ein Stab ist.

5. System (100) nach Anspruch 1, wobei das anatomische Element ein Wirbel ist und der Marker (126) lösbar an einem Schraubenkopf einer in den Wirbel eingebetteten Schraube befestigt ist.

6. System (100) nach Anspruch 5, wobei der Schraubenkopf einen Gewindehohlraum umfasst, der konfiguriert ist, um einen Gewindevorsprung des Markers (126) aufzunehmen.

7. System (100) nach Anspruch 6, wobei der Speicher (106) weitere Daten zum Verarbeiten durch den Prozessor (104) speichert, die, wenn sie verarbeitet werden, den Prozessor (104) veranlassen zum:
Vergleichen der Pose des Markers (126) mit einer vorbestimmten Pose; und
Validieren der Pose des Markers (126), wenn die Pose im Wesentlichen mit der vorbestimmten Pose übereinstimmt.

8. System (100) nach Anspruch 1, wobei die Pose mindestens eine von einer ersten Pose des Markers (126) und einer zweiten Pose des
Markers (126) umfasst.

9. System (100) nach Anspruch 8, wobei das Erfassen einer Bewegung des Markers (126) das Vergleichen der ersten Pose des Markers (126) und der zweiten Pose des Markers (126) umfasst.

10. System (100) nach Anspruch 1, wobei die Pose von mindestens einem von einem Navigationssystem (118), das konfiguriert ist, um eine Pose des Markers (126) zu verfolgen, oder einem Roboterarm (116), der den Marker (126) orientiert, erhalten wird, wobei der Roboterarm (116) mindestens einen Sensor (128) zum Erfassen einer Pose des Roboterarms (116) umfasst.

## Revendications

1. Système (100) permettant de suivre le mouvement d'un élément anatomique, le système comprenant :
un marqueur (126) couplé à l'élément anatomique ;
un processeur (104) ; et
une mémoire (106) stockant des données destinées à être traitées par le processeur (104), les données, lorsqu'elles sont traitées, amènent le processeur (104) à :
suivre le marqueur (126) ;
détecter un mouvement du marqueur (126) ;
déterminer une position du marqueur (126) en fonction du mouvement ; et
recevoir une première image de l'élément anatomique, la première image étant obtenue en préopératoire,
**caractérisé en ce que**
la mémoire (106) stocke des données supplémentaires destinées à être traitées par le processeur (104) qui, lorsqu'elles sont traitées, amènent le processeur (104) à :
obtenir une seconde image à partir d'un dispositif d'imagerie (112), la seconde image représentant le marqueur (126) et l'élément anatomique ; et
mettre à jour la première image de l'élément anatomique en fonction de la seconde image (126) de l'élément anatomique et du mouvement détecté.

2. Système (100) selon la revendication 1, dans lequel le marqueur (126) comprend au moins un parmi un marqueur optique (126), un traceur électromagnétique, un traceur d'identification par radiofréquence, un marqueur magnétique, une diode électroluminescente ou une diode électroluminescente infrarouge.

3. Système (100) selon la revendication 1, dans lequel le marqueur (126) est intégré à un implant chirurgical, l'implant chirurgical étant fixé à l'élément anatomique.

4. Système (100) selon la revendication 3, dans lequel l'implant chirurgical est une tige.

5. Système (100) selon la revendication 1, dans lequel l'élément anatomique est une vertèbre et le marqueur (126) est fixé de manière amovible à une tête de vis d'une vis intégrée dans la vertèbre.

6. Système (100) selon la revendication 5, dans lequel la tête de vis comprend une cavité filetée conçue pour recevoir une saillie filetée du marqueur (126).

7. Système (100) selon la revendication 6, dans lequel la mémoire (106) stocke des données supplémentaires destinées à être traitées par le processeur (104) qui, lorsqu'elles sont traitées, amènent le processeur (104) à :
comparer la position du marqueur (126) à une position prédéterminée ; et
valider la position du marqueur (126) lorsque la position correspond sensiblement à la position prédéterminée.

8. Système (100) selon la revendication 1, dans lequel la position comprend au moins l'une parmi une première position du marqueur (126) et une seconde position du marqueur (126).

9. Système (100) selon la revendication 8, dans lequel la détection du mouvement du marqueur (126) comprend la comparaison de la première position du marqueur (126) et de la seconde position du marqueur (126).

10. Système (100) selon la revendication 1, dans lequel la position est obtenue à partir d'au moins l'un parmi un système de navigation (118) configuré pour suivre une position du marqueur (126) ou un bras robotisé (116) orientant le marqueur (126), le bras robotisé (116) comprenant au moins un capteur (128) pour détecter une position du bras robotisé (116).
